# EUROPÄISCHE PATENTSCHRIFT

(11) **EP 0 835 613 B1**
(45) Veröffentlichungstag und Bekanntmachung des Hinweises auf die Patenterteilung: **22.01.2003**
(21) Anmeldenummer: 97117759.7
(22) Anmeldetag: 14.10.1997
(51) Int. Cl.: A23L 1/00, A61K 9/107, A23L 1/302, A23K 1/16

(54) **Stabile Emulsionen und Trockenpulver von Mischungen fettlöslicher Vitamine, deren Herstellung und Verwendung**
Stable emulsions and dry powder from mixtures of fat-soluble vitamins, their process of preparation and their use
Emulsions stables et poudre sèche de vitamines liposolubles, leur procédé de préparation et leur utilisation

(30) Priorität: 14.10.1996 DE 19642359
(43) Veröffentlichungstag der Anmeldung: 15.04.1998
(73) Patentinhaber: BASF AKTIENGESELLSCHAFT, 67056 Ludwigshafen (DE)
(72) Erfinder: Hähnlein, Wolfgang, Dr., 67251 Freinsheim (DE); Hansen, Morten Mohr, Kopenhagen (DK); Olesen, Jes Elenius, Kopenhagen (DK); Tobiasen, Anne Grethe, Snekkersten (DK)

(56) Entgegenhaltungen:
- FR-A- 2 355 462
- US-A- 4 966 779
- DATABASE WPI Section Ch, Week 9742 Derwent Publications Ltd., London, GB; Class B04, AN 97-449398 XP002054795 & CN 1 116 925 A (WANG W) , 21.Februar 1996
- DATABASE WPI Section Ch, Week 8344 Derwent Publications Ltd., London, GB; Class A96, AN 83-805364 XP002054796 & JP 58 162 517 A (GREEN CROSS CORP) , 27.September 1983

## Beschreibung

Die vorliegende Erfindung betrifft neue stabile Emulsionen und Trockenpulver von Mischungen fettlöslicher Vitamine sowie ein Verfahren zu deren Herstellung und ihre Verwendung.

Fettlösliche Vitamine spielen eine bedeutende Rolle in der menschlichen und tierischen Ernährung. Gemeinsam ist diesen fettlöslichen Wirkstoffen, daß sie in ihrer reinen Form nur schwer oder gar nicht handhabbar sind, da es sich um oxidationsempfindliche Substanzen handelt. Des weiteren ist für eine optimale Resorbierbarkeit und somit Bioverfügbarkeit eine Feinverteilung des Wirkstoffs von Vorteil. Deshalb werden diese Stoffe häufig in Form von Emulsionen oder bevorzugt in Form von Trockenpulvern angeboten, wobei die Wirkstoffe entweder in reiner Form oder als Lösung in einem physiologisch verträglichen Öl feinverteilt in einem Schutzkolloid eingebettet sind.

Auf Basis literaturbekannter Formulierverfahren, wie sie u.a. in R.A. Morton, Fat Soluble Vitamins, Intern. Encyclopedia of Food and Nutrition, Bd. 9, Pergamon Press 1970, S. 134-139, zusammenfassend beschrieben sind, lassen sich Trockenpulver von fettlöslichen Vitaminen herstellen. Dabei wird das fettlösliche Vitamin in eine wäßrige Schutzkolloid-Lösung einemulgiert, homogenisiert und anschließend, gegebenenfalls in Gegenwart eines Überzugs(Coating)-Materials, getrocknet.

Als Schutzkolloide werden häufig die in den Patentschriften DE-A-1 123 084, US 2756177, US 4670247 sowie in EP-A-0 285 682, EP-A-0 565 989 und DE-A-44 24 085 beschriebenen Biopolymere wie Gelatine, Gummi Arabicum, Stärke, Lignin oder auch teilhydrolysiertes Sojaprotein sowie andere aus natürlichen Quellen gewonnene Stoffe verwendet. Eine Zusammenfassung handelsüblicher Schutzkolloide ist ebenfalls aus R.A. Morton, Fat Soluble Vitamins, Intern. Encyclopedia of Food and Nutrition, Bd. 9, Pergamon Press 1970, S. 130/131, zu entnehmen.

Bei der Verwendung von Trockenpulvern, die Mischungen fettlöslicher Vitamine enthalten, deren Zusammensetzung dem physiologischen Bedarf angepaßt ist und bei denen die Einzelkomponenten teilweise im extremen Über- bzw. Unterschuß vorliegen, werden besonders hohe Anforderungen an die Konfektionierung gestellt. Für den Anwender ist es in diesem Fall besonders wichtig, daß neben der gewünschten Stabilität in allen Partikeln eine homogene Gleichverteilung der Wirkstoffe gewährleistet ist.

Es bestand daher die Aufgabe, ein Verfahren zur Herstellung stabiler Mischungen von fettlöslichen Vitaminen in feinverteilter, Form vorzuschlagen. Ferner sollten Trockenpulver von fettlöslichen Vitaminen zur Verfügung gestellt werden, bei denen neben der gewünschten Stabilität, eine homogene Gleichverteilung der Wirkstoffe in allen Partikeln gewährleistet ist.

Der Begriff "fettlösliche Vitamine" umfaßt im Rahmen der vorliegenden Erfindung insbesondere die Vitamine A, D, E und/oder K, die entsprechenden Provitamine und Vitamin-Derivate wie z.B. Ester mit Vitamin A-, D-, E- oder K-artiger Wirkung, wobei der in den Ansprüchen formulierte Ausdruck "Vitamin (A,D,E,K)" ebenso deren Derivate wie auch eine Mischung derselben beinhaltet.

Die Aufgabe wurde erfindungsgemäß gelöst mit einem Verfahren zur Herstellung einer stabilen Emulsion oder eines stabilen Trockenpulvers von fettlöslichen Vitaminen dadurch gekennzeichnet, daß man jeweils separat in Wasser emulgiert:
a) Vitamin K und
b) eine Mischung aus Vitamin D, Vitamin E sowie Vitamin A oder einem inerten Öl oder eine Mischung aus Vitamin A und einem inerten Öl, erhältlich, indem man
   b₁) Vitamin D in einem inerten Öl und/oder Vitamin A löst und mit Vitamin E mischt oder
   b₂) eine Mischung aus Vitamin E und Vitamin A und/oder einem inerten Öl mit Vitamin D versetzt,
   wobei man das Emulgieren der Komponenten a) und b) jeweils in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe,
vornimmt, anschließend die Emulsionen mischt und die Mischung für die Herstellung eines stabilen Vitamin-Trockenpulvers, gegebenenfalls in Gegenwart eines Überzugsmaterials, trocknet.

Erfolgt die Formulierung der Vitaminmischung gemäß den literaturbekannten Verfahren, nach denen zunächst die fettlöslichen Vitamine gemischt, anschließend in eine wäßrige Schutzkolloid-Lösung einemulgiert, homogenisiert und zuletzt getrocknet werden, so sind folgende Phänomene zu beobachten:

Eine vor dem Emulgierschritt hergestellte ölige Mischung aus Vitamin K mit Vitamin A und/oder Vitamin E färbt sich spontan tiefrot. Diese Rotfärbung ist aus anwendungstechnischer Sicht in der Regel nicht gewünscht.

Des weiteren ist festzustellen, daß eine Mischung aus Vitamin E und Vitamin D, in der Vitamin E einen Anteil von über 70 Gew.% ausmacht, zu einem massiven Abbau von Vitamin D führt. So wurde beispielsweise bei einer Mischung aus 99.6 Gew.-Teilen Vitamin E und 0.4 Gew.-Teilen Vitamin D ein Aktivitätsverlust an Vitamin D von rund 50% gemessen.

Mit Hilfe des erfindungsgemäßen Verfahrens lassen sich nun die Stabilitätsprobleme beseitigen sowie die Farbe des Trockenpulvers gezielt steuern. So bleibt durch separates Emulgieren von Vitamin K einerseits und dem Gemisch von Vitamin A, D, und/oder E andererseits, die Eigenfärbung der Vitamine erhalten. Selbst die anschließende Mischung der beiden hergestellten Emulsionen behält weiterhin ihre helle Gelbfärbung.

Die überraschenden Aktivitätsverluste von Vitamin D in Gegenwart großer Mengen an Vitamin E konnten erfindungsgemäß dadurch vermieden werden, daß man Vitamin D zunächst in Vitamin A und/oder einem inerten Öl löst und die erhaltene Lösung anschließend mit Vitamin E versetzt, wobei die Gew.-Teile an Vitamin A und/oder dem inerten Öl mindestens 25%, bevorzugt größer 50%, insbesondere mehr als 100% der Vitamin E Gew.-Teile betragen. Entsprechend einer weiteren Verfahrensvariante zur Stabilisierung von Vitamin D wird die Konzentration von Vitamin E zunächst durch "Verdünnen" mit mindestens 25 Gew.%, bevorzugt mehr als 50 Gew.% an Vitamin A und/oder einem inerten Öl erniedrigt, um anschließend diese Mischung mit Vitamin D zu versetzen.

Für bestimmte Anwendungsgebiete z.B. zum Färben von Lebensmitteln oder Pharmazeutika ist eine stabile Formulierung der o.g. rotgefärbten Vitaminmischung vorteilhaft. Ein solches Produkt erhält man mittels eines erfindungsgemäßen Verfahrens, das dadurch gekennzeichnet ist, daß man eine Mischung aus
a) Vitamin D, Vitamin E und Vitamin K sowie Vitamin A oder einem inerten Öl oder eine Mischung aus Vitamin A und einem inerten Öl, erhältlich, indem man
   a₁) Vitamin D in einem inerten Öl und/oder Vitamin A löst und mit Vitamin E und Vitamin K mischt oder
   a₂) eine Mischung aus Vitamin E und Vitamin K sowie Vitamin A und/oder einem inerten Öl mit Vitamin D versetzt,
   in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe
in wasser emulgiert und die Emulsion für die Herstellung eines stabilen Vitamin-Trockenpulvers, gegebenenfalls in Gegenwart eines Überzugsmaterials, trocknet.

Während auch diese Herstellvariante durch die Verfahrensschritte a₁) oder a₂) die Stabilität von Vitamin D gewährleistet, wird durch die Mischung von Vitamin E mit Vitamin K vor der Emulgierung ein dunkelrot gefärbtes Öl erzeugt, dessen Farbe die spätere Emulsion sowie das nach der Trocknung erhaltene Trockenpulver kennzeichnet.

Die Mischung der fettlöslichen Vitamine, die in den beiden erfindungsgemäßen Verfahrensvarianten eingesetzt werden, setzt sich zusammen aus 5 bis 90 Gew.% Vitamin A und/oder einem inerten Öl, 5 bis 90 Gew.% Vitamin E, 0.01 bis 1 Gew.% Vitamin D und 0.1 bis 10 Gew.% Vitamin K, wobei sich die Gew.%-Angaben der Einzelkomponenten zu 100% addieren.

Eine besondere Ausführungsform der oben genannten Verfahren ist dadurch gekennzeichnet, daß das Vitamingemisch 25 bis 70 Gew.% Vitamin A und/oder ein inertes Öl, 30 bis 70 Gew. % Vitamin E, 0.05 bis 0.4 Gew.% Vitamin D und 2 bis 8 Gew.% Vitamin K enthält, wobei sich die Gew.%-Angaben der Einzelkomponenten zu 100% addieren. Führen die beiden o.g. erfindungsgemäßen Verfahren zu stabilen Emulsionen und Trockenpulvern von Mischungen der fettlöslichen Vitamine A, D, E und K sowie der Vitamine D, E und K, so galt es, auch Lösungen für die Stabilitätsproblematik der folgenden Dreierkombinationen, nämlich der Vitamine A, D, K; der Vitamine A, E, K sowie der Vitamine A, D, E zu finden.

Diese Aufgabe wurde durch folgende Verfahren zur Herstellung stabiler Vitamin-Emulsionen und Trockenpulver gelöst, die dadurch gekennzeichnet sind, daß man
1) für die Vitamin A, D und K Dreierkombination jeweils separat in Wasser emulgiert:
   a) Vitamin K und
   b) eine Mischung aus Vitamin D und Vitamin A, wobei man das Emulgieren der Komponenten a) und b) jeweils in Gegenwart
   c) eines oder mehrerer Schutzkolloide,
   d) gegebenenfalls eines oder mehrerer Zucker und/oder Zuckeralkohole und
   e) gegebenenfalls weiterer Zusatzstoffe
   vornimmt, anschließend die Emulsionen mischt und die Mischung für die Herstellung eines stabilen Vitamin-Trockenpulvers, gegebenenfalls in Gegenwart eines Überzugsmaterials, trocknet.
2) für die Vitamin A, D und K Dreierkombination:
   a) eine Mischung aus Vitamin A, Vitamin K und Vitamin D in Gegenwart
   c) eines oder mehrerer Schutzkolloide,
   d) gegebenenfalls eines oder mehrerer Zucker und/oder Zuckeralkohole und
   e) gegebenenfalls weiterer Zusatzstoffe in Wasser emulgiert
   und die Emulsion für die Herstellung eines stabilen Vitamin-Trockenpulvers, gegebenenfalls in Gegenwart eines Überzugsmaterials, trocknet.
3) für die Vitamin A, E und K Dreierkombination jeweils separat in Wasser emulgiert:
   a) Vitamin K und
   b) eine Mischung aus Vitamin A und Vitamin E,
      wobei man das Emulgieren der Komponenten a) und b) jeweils in Gegenwart
   c) eines oder mehrerer Schutzkolloide,
   d) gegebenenfalls eines oder mehrerer Zucker und/oder Zuckeralkohole und
   e) gegebenenfalls weiterer Zusatzstoffe,
   vornimmt, anschließend die Emulsionen mischt und die Mischung für die Herstellung eines stabilen Vitamin-Trockenpulvers, gegebenenfalls in Gegenwart eines Überzugsmaterials, trocknet.
4) für die Vitamin A, E und K Dreierkombination:
   a) eine Mischung aus Vitamin A, Vitamin E und Vitamin K in Gegenwart
   c) eines oder mehrerer Schutzkolloide,
   d) gegebenenfalls eines oder mehrerer Zucker und/oder Zuckeralkohole und
   e) gegebenenfalls weiterer Zusatzstoffe
   in Wasser emulgiert und die Emulsion für die Herstellung eines stabilen Vitamin-Trockenpulvers, gegebenenfalls in Gegenwart eines Überzugsmaterials, trocknet.
5) für eine Mischung aus
   a) Vitamin D, Vitamin E sowie Vitamin A oder einem inerten Öl oder eine Mischung aus Vitamin A und einem inerten Öl, erhältlich, indem man
      a₁) Vitamin D in einem inerten Öl und/oder Vitamin A löst und mit Vitamin E mischt oder
      a₂) eine Mischung aus Vitamin E und Vitamin A und/oder einem inerten Öl mit Vitamin D versetzt,
      in Gegenwart
   c) eines oder mehrerer Schutzkolloide,
   d) gegebenenfalls eines oder mehrerer Zucker und/oder Zuckeralkohole und
   e) gegebenenfalls weiterer Zusatzstoffe
   in Wasser emulgiert und die Emulsion für die Herstellung eines stabilen Vitamin-Trockenpulvers, gegebenenfalls in Gegenwart eines Überzugsmaterials, trocknet.

Die Mischung der fettlöslichen Vitamine, die in den erfindungsgemäßen Verfahren 1) und 2) eingesetzt wird, setzt sich zusammen aus 70 bis 98 Gew.% Vitamin A, 0.01 bis 1 Gew.% Vitamin D und 2 bis 30 Gew.% Vitamin K.

In den Verfahren 3) und 4) werden Mischungen fettlöslicher Vitamine eingesetzt, die wie folgt zusammengesetzt sind: 5 bis 90 Gew.% Vitamin A, 5 bis 90 Gew.% Vitamin E und 0.1 bis 10 Gew.% Vitamin K.

Die Vitaminmischung, die im erfindungsgemäßen Verfahren 5) eingesetzt wird, setzt sich zusammen aus 5 bis 90 Gew.% Vitamin A und/oder eines inerten Öls, 5 bis 90 Gew.% Vitamin E und 0.01 bis 1 Gew.% Vitamin D, wobei sich die Gew.%-Angaben der jeweiligen o.g. Einzelkomponenten zu 100% addieren.

Alle fünf der oben aufgeführten Verfahrensvarianten zur Herstellung von Emulsionen und Trockenpulvern fettlöslicher Vitamine, die definitionsgemäß eine Dreierkombination aus der Gruppe der Vitamine A, D, E und K beinhalten, führen zu stabilen Produkten, deren Farbe durch die jeweiligen erfindungsgemäßen Emulgierverfahren gezielt eingestellt werden kann.

Bei der Durchführung der erfindungsgemäßen Verfahren werden im ersten Verfahrensschritt die fettlöslichen Vitamine gemischt, wobei im Falle der Vitaminkombinationen A, D, E, K; D, E, K; A, D, E sowie D und E zunächst die Herstellung einer Lösung von Vitamin D in Vitamin A und/oder einem inerten Öl bei höherer Temperatur, z.B. bei einer Temperatur zwischen ca. 40°C bis 80°C, bevorzugt 50°C bis 70°C erfolgt. Diese Lösung wird danach mit den restlichen Vitaminen versetzt und bei 50°C bis 70°C in eine wäßrige Schutzkolloid-Lösung einemulgiert.

Es ist aber auch möglich, zunächst Vitamin E mit Vitamin A, Vitamin K und/oder einem inerten Öl bei den o. g. Temperaturen zu mischen, um somit die Konzentration an Vitamin E zu verringern und erst diese Mischung mit Vitamin D zu versetzen und sie anschließend in eine wäßrige Schutzkolloid-Lösung zu emulgieren.

In den Fällen, in denen bei Anwesenheit von Vitamin K eine Rotfärbung der Vitamin-Emulsion nicht wünschenswert ist, ist es vorteilhaft, sowohl Vitamin K als auch die Mischung aus den Vitaminen A, E und/oder D und gegebenenfalls einem inerten Öl jeweils separat unter den o. g. Bedingungen zu emulgieren und diese Emulsionen anschließend zu mischen.

Nach Homogenisierung und Einstellung der Viskosität der Emulsion(en) auf 50 bis 300 cP, bevorzugt 70 bis 150 cP, durch entsprechende Verdünnung mit Wasser, kann die Überführung der Emulsion(en) in ein pulverförmiges Präparat in an sich bekannter Weise erfolgen, z.B. durch Sprühtrocknung oder durch Sprühkühlung oder durch Versprühen der Emulsion in einem Sprühturm unter Mitverwendung eines inerten Überzugs-(Coating)-Materials, Auffangen der umhüllten Teilchen und Trocknen im Wirbelbett.

Die nach den erfindungsgemäßen Verfahren hergestellten stabilen Vitamin-Trockenpulver enthalten 3 bis 40 Gew.% eines Gemisches aus Vitamin A, Vitamin D, Vitamin E und/oder Vitamin K und/oder einem inerten Öl, 5 bis 40 Gew.% eines Schutzkolloids, 0 bis 30 Gew.% eines Zuckers und/oder Zuckeralkohols, 0 bis 70 Gew.% eines Überzugsmaterials und gegebenenfalls 0 bis 25 Gew.% weiterer Zusatzstoffe, wobei sich die Gew.%-Angaben der Einzelkomponenten zu 100% addieren.

Unter der Bezeichnung "inertes Öl" sind physiologisch zugelassene Öle, wie beispielsweise Sesamöl, Maiskeimöl, Baumwollsaatöl, Kokosnußöl, Sojabohnenöl oder Erdnußöl sowie Ester mittelkettiger pflanzlicher Fettsäuren zu verstehen.

Als Schutzkolloide werden beispielsweise Gelatine, Fischgelatine, Stärke, Dextrin, Pflanzenproteine, Pektin, Gummi-Arabikum, Kasein, Kaseinat oder Mischungen davon verwendet, wobei Gelatine, Gummi arabicum, Pflanzenproteine und/oder modifizierte Stärke bevorzugt eingesetzt werden. Es sind aber auch Polyvinylalkohol, Polyvinylpyrrolidon, Methylcellulose, Carboxymethylcellulose, Hydroxypropylcellulose und Alginate als mögliche Schutzkolloide denkbar. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen. Zur Erhöhung der mechanischen Stabilität des Endproduktes ist es zweckmäßig, dem Kolloid einen Weichmacher zuzusetzen, wie Zucker oder Zuckeralkohole, z.B. Saccharose, Glucose, Fructose, Laktose, Invertzucker, Sorbit, Mannit, Maltodextrin oder Glycerin. In bevorzugten Ausführungsformen des erfindungsgemäßen Verfahrens werden Saccharose, Laktose und/oder Maltodextrin eingesetzt.

Als weitere Zusatzstoffe können zur Erhöhung der Stabilität der Wirkstoffe gegen oxidativen Abbau Stabilisatoren wie t-Butylhydroxy-toluol, t-Butylhydroxyanisol, Ascorbinsäure oder Ethoxyquine zugesetzt werden. Vorzugsweise werden sie gemeinsam mit den Vitaminen und gegebenenfalls zusätzlichen Emulgatoren in die wäßrige Schutzkolloid-Lösung einemulgiert. Als Emulgatoren können beispielsweise Ascorbylpalmitat, Polyglycerin-Fettsäureester, Sorbitan-Fettsäureester, Propylenglycol-Fettsäureester oder Lecithin verwendet werden.

Unter dem Begriff Überzugs- oder auch Coatingmaterial versteht man Verbindungen wie Stärke und/oder Stärkederivate sowie Kieselsäure und/oder Kieselsäurederivate, mit deren Hilfe die Vitamin Trockenpulver physikalisch stabilisiert werden. Bevorzugte Überzugsmaterialien im Lebensmittelbereich sind Stärke und Stärkederivate, insbesondere Mais- oder Reisstärke.

Die erfindungsgemäßen Präparate können sowohl als Zusatz zu Lebensmitteln und Pharmazeutika als auch für die Tierernährung Verwendung finden. In gewissen Fällen kann es auch zweckmäßig sein, die hergestellten Emulsionen nicht erst in Trockenpulver überzuführen, sondern direkt als solche zu verwenden.

In den nachfolgenden Beispielen wird die Durchführung der erfindungsgemäßen Verfahren näher erläutert.

### Beispiel 1:

### a) Herstellung der Vitamin-Mischung:

1,7 g (40 mio. IU/g) Vitamin D₃ wurden bei 65°C unter Rühren in 500 g (1,7 mio. IU/g) vorgewärmtes Vitamin A-Palmitat gelöst und anschließend mit 500 g (1.100 IU/g) d,1-α-Tocopherol und 45 g Vitamin K₁ bei 65°C versetzt.

### b) Herstellung der Emulsion:

2 kg Gummi Arabicum und 2 kg Saccharose wurden bei 65°C in einem Emulgierbehälter in 9 kg destilliertem Wasser gelöst. Nach 30 minütigem Rühren wurde die Vitamin-Mischung hinzugegeben und das Gemisch für weitere 2 h homogenisiert. Anschließend wurde die Emulsion mit Wasser bis zu einer Viskosität von ca. 100 cP verdünnt, in einem Sprühturm in Stärke eingehüllt und getrocknet.

Man erhielt ein gelbbraun gefärbtes Vitamin-Trockenpulver mit folgenden Vitamingehalt:

| | |
|---|---|
| Vitamin A-Palmitat | 91.000 IU/g |
| Vitamin D₃ | 7090 IU/g |
| Vitamin K₁ | 0,41% |
| d,1-α-Tocopherol | 4,88% |

### Beispiel 3:

### a) Herstellung der Vitamin-Mischung:

1,7 g (40 mio. IU/g) Vitamin D₃ wurden unter Rühren in 500 g (1,7 miö. IU/g) 65°C warmen Vitamin A-Palmitat gelöst und anschließend mit 500 g (1.100 IU/g) d,1-α-Tocopherol versetzt.

### b) Herstellung der Emulsion:

2 kg Gummi Arabicum und 2 kg Saccharose wurden bei 65°C in einem Emulgierbehälter in 9 kg destilliertem Wasser gelöst. Nach 30 minütigem Rühren wurden 500 g der Lösung in einen 1 l Reaktor überführt und mit 45 g Vitamin K₁ homogenisiert. Die restliche Schutzkolloid-Lösung wurde mit der Vitamin-Mischung aus a) versetzt und ca. 2 h homogenisiert. In einem separaten Emulgierbehälter wurden die beiden Emulsionen langsam gemischt, mit Wasser bis zu einer Viskosität von ca. 100 cP verdünnt, in einem Sprühturm in Stärke eingehüllt und getrocknet.

Das hellgelb gefärbte Vitamin-Trockenpulver hatte folgenden Vitamingehalt:

| | |
|---|---|
| Vitamin A-Palmitat | 103.000 IU/g |
| Vitamin D₃ | 7540 IU/g |
| Vitamin K₁ | 0,49% |
| d,1-α-Tocopherol | 4,68% |

### Beispiel 4:

### a) Herstellung der Vitamin-Mischung:

1,7 g (40 mio. IU/g) Vitamin D₃ wurden unter Rühren in 65 g fraktioniertes Kokosnußöl gelöst und anschließend mit einer 65°C warmen Mischung aus 500 g (1,7 mio. IU/g) Vitamin A-Palmitat und 500 g (1.100 IU/g) d,1-α-Tocopherol versetzt.

### b) Herstellung der Emulsion:

2 kg Gummi Arabicum und 2 kg Saccharose wurden bei 65°C in einem Emulgierbehälter in 9 kg destilliertem Wasser gelöst. Nach 30 minütigem Rühren wurden 500 g der Lösung in einen 1 l Reaktor überführt und mit 45 g Vitamin K₁ homogenisiert. Die restliche Schutzkolloid-Lösung wurde mit der Vitamin-Mischung aus a) versetzt und ca. 2 h homogenisiert. In einem separaten Emulgierbehälter wurden die beiden Emulsionen langsam gemischt, mit Wasser bis zu einer Viskosität von ca. 100 cP verdünnt, in einem Sprühturm in Stärke eingehüllt und getrocknet.

Das hellgelb gefärbte Vitamin-Trockenpulver hatte folgenden Vitamingehalt:

| | |
|---|---|
| Vitamin A-Palmitat | 71.600 IU/g |
| Vitamin D₃ | 5850 IU/g |
| Vitamin K₁ | 0,36% |
| d,1-α-Tocopherol | 3,96% |

### Beispiel 5:

### a) Herstellung der Vitamin-Mischung:

1,2 g (40 mio. IU/g) Vitamin D₃ wurden unter Rühren in 350 g (1,7 mio. IU/g) Vitamin A-Palmitat bei 65°C gelöst und anschließend mit 350 g (1.100 IU/g) d,1-α-Tocopherol versetzt.

### b) Herstellung der Emulsion:

1,4 kg mit Natrium Octenyl-Succinat modifizierte Stärke und 1,4 kg Saccharose wurden bei 65°C in einem Emulgierbehälter in 6,3 kg destilliertem Wasser gelöst. Nach 30 minütigem Rühren wurden 500 g der Lösung in einen 1 l Reaktor überführt und mit 33 g Vitamin K₁ homogenisiert. Die restliche Schutzkolloid-Lösung wurde mit der Vitamin-Mischung aus a) versetzt und ca. 2 h homogenisiert. In einem separaten Emulgierbehälter wurden die beiden Emulsionen langsam gemischt, mit Wasser bis zu einer Viskosität von ca. 100 cP verdünnt und anschließend in einem Sprühturm in Stärke eingehüllt und getrocknet.

Das hellgelb gefärbte Vitamin-Trockenpulver hatte folgenden Vitamingehalt:

| | |
|---|---|
| Vitamin A-Palmitat | 85.300 IU/g |
| Vitamin D₃ | 7080 IU/g |
| Vitamin K₁ | 0,44% |
| d,1-α-Tocopherol | 4,78% |

### Beispiel 6:

### a) Herstellung der Vitamin-Mischung:

2,6 g (40 mio. IU/g) Vitamin D₃ wurden unter Rühren bei 65°C in 750 g (1,7 mio. IU/g) vorgewärmtes Vitamin A-Palmitat gelöst und anschließend mit 750 g (1.100 IU/g) d,1-α-Tocopherol bei 65°C versetzt.

### b) Herstellung der Emulsion:

2 kg Natrium Caseinat und 4 kg Saccharose wurden bei 65°C in einem Emulgierbehälter in 7 kg destilliertem Wasser gelöst. Nach 30 minütigem Rühren wurden 500 g der Lösung in einen 1 l Reaktor überführt und mit 68 g Vitamin K₁ homogenisiert. Die restliche Schutzkolloid-Lösung wurde mit der Vitamin-Mischung aus a) versetzt und ca. 2 h homogenisiert. In einem separaten Emulgierbehälter wurden die beiden Emulsionen langsam gemischt, mit Wasser bis zu einer Viskosität von ca. 100 cP verdünnt, in einem Sprühturm in Stärke eingehüllt und getrocknet.

Das hellgelb gefärbte Vitamin-Trockenpulver hatte folgenden Vitamingehalt:

| | |
|---|---|
| Vitamin A-Palmitat | 86.500 IU/g |
| Vitamin D₃ | 6950 IU/g |
| Vitamin K₁ | 0,47% |
| d,1-α-Tocopherol | 4,8% |

### Beispiel 7 (Vergleichsbeispiel)

### Herstellung der Vitamin-Mischung:

1,7 g (40 mio. IU/g) Vitamin D₃ wurden unter Rühren bei 65°C in 500 g (1.100 IU/g) d,1-α-Tocopherol gelöst und anschließend mit 500 g (1,7 mio. IU/g) 65°C warmen Vitamin A-Palmitat versetzt.

Anschließend wurde das Gemisch auf 4°C abgekühlt und bei dieser Temperatur für einen Zeitraum von 2 Wochen aufbewahrt. Nach 4 bzw. 10 Tagen wurden Gehaltsbestimmungen der Vitamine in dem Gemisch durchgeführt.

| 4 Tage | | |
|---|---|---|
| | berechnet | gemessen |
| Vitamin A-Palmitat | 850.000 IU/g | 848.000 IU/g |
| Vitamin D₃ | 66.000 IU/g | 33.500 IU/g |
| d,1-α-Tocopherol | 49,9% | 50,0% |
| | | |

| 10 Tage | | |
|---|---|---|
| | berechnet | gemessen |
| Vitamin D₃ | 66.000 IU/g | 33.500 IU/g |

### Beispiel 8:

Die in Tabelle 1 aufgeführten Mengen an Vitamin D₃ (40 mio. IU/g) oder einer Lösung von Vitamin D₃ in fraktioniertem Kokosnußöl wurden unter Rühren bei 65°C mit verschiedenen Mengen d,1-α-Tocopherol (1.100 IU/g) gemischt. Eine Probe der Mischung wurde direkt nach der Zubereitung auf ihren Vitamin D-Gehalt analysiert. Von der restlichen Vitamin-Mischung wurden je eine Hälfte 24 Stunden bei 65°C und die zweite Hälfte 24 Stunden bei 5°C aufbewahrt. Beide Hälften wurden anschließend auf ihren Vitamin D₃-Gehalt hin untersucht.

**Tabelle 1:**

| Versuch | Vitamin D [mg] | Kokosöl [g] | Vitamin E [g] | Gehalt Vit. D (5 min, 65°C) [% der Th.] | Gehalt Vit. D (24 h, 65°C) [% der Th.] | Gehalt Vit. D (24 h, 5°C) [% der Th.] |
|---|---|---|---|---|---|---|
| 1 | 52,9 | | 14,90 | 69 | 2 | 61 |
| 2 | 52,3 | 3,68 | 10,90 | 89 | 50 | 90 |
| 3 | 51,1 | 7,28 | 7,30 | 93 | 87 | 93 |
| 4 | 54,9 | 10,99 | 3,68 | 94 | 86 | 90 |

## Patentansprüche

1. Verfahren zur Herstellung einer stabilen Vitamin-Emulsion oder eines stabilen Vitamin-Trockenpulvers **dadurch gekennzeichnet, daß** man bei 50°C bis 70°C jeweils separat in Wasser emulgiert:
a) Vitamin K und
b) eine Mischung aus Vitamin D, Vitamin E sowie Vitamin A oder einem inerten Öl oder eine Mischung aus Vitamin A und einem inerten Öl, erhältlich, indem man
b₁) Vitamin D in einem inerten Öl und/oder Vitamin A bei einer Temperatur zwischen 40°C bis 80°C löst und mit Vitamin E mischt oder
b₂) eine Mischung aus Vitamin E und Vitamin A und/oder einem inerten Öl mit Vitamin D versetzt,
wobei man das Emulgieren der Komponenten a) und b) jeweils in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe,
vornimmt, anschließend die Emulsionen mischt, homogenisiert und durch Verdünnung mit Wasser die Viskosität der Emulsionen auf 50 bis 300 cP einstellt und die Mischung für die Herstellung eines stabilen Vitamin-Trockenpulvers, gegebenenfalls in Gegenwart eines Überzugsmaterials, trocknet.

2. Verfahren zur Herstellung einer stabilen Vitamin-Emulsion oder eines stabilen Vitamin-Trockenpulvers **dadurch gekennzeichnet, daß** man eine Mischung aus
a) Vitamin D, Vitamin E und Vitamin K sowie Vitamin A oder einem inerten Öl oder eine Mischung aus Vitamin A und einem inerten Öl, erhältlich, indem man
a₁) Vitamin D in einem inerten Öl und/oder Vitamin A bei einer Temperatur zwischen 40°C bis 80°C löst und mit Vitamin E und Vitamin K mischt oder
a₂) eine Mischung aus Vitamin E und Vitamin K sowie Vitamin A und/oder einem inerten Öl mit Vitamin D versetzt,
in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe
in Wasser bei 50°C bis 70°C emulgiert, die Emulsion homogenisiert und durch Verdünnung mit Wasser die Viskosität der Emulsionen auf 50 bis 300 cP einstellt und für die Herstellung eines stabilen Vitamin-Trockenpulvers, gegebenenfalls in Gegenwart eines Überzugsmaterials, trocknet.

3. Verfahren nach den Ansprüchen 1 und 2 **dadurch gekennzeichnet, daß** das Vitamingemisch 5 bis 90 Gew.% Vitamin A und/oder ein inertes Öl, 5 bis 90 Gew.% Vitamin E, 0.01 bis 1 Gew.% Vitamin D und 0.1 bis 10 Gew.% Vitamin K enthält, wobei sich die Gew.%-Angaben der Einzelkomponenten zu 100% addieren.

4. Verfahren nach den Ansprüchen 1 bis 3 **dadurch gekennzeichnet, daß** das Vitamingemisch 25 bis 70 Gew.% Vitamin A und/oder ein inertes Öl, 30 bis 70 Gew. % Vitamin E, 0.05 bis 0.4 Gew.% Vitamin D und 2 bis 8 Gew.% Vitamin K enthält, wobei sich die Gew.%-Angaben der Einzelkomponenten zu 100% addieren.

5. Verfahren zur Herstellung einer stabilen Vitamin-Emulsion oder eines stabilen Vitamin-Trockenpulvers **dadurch gekennzeichnet, daß** man bei 50°C bis 70°C jeweils separat in Wasser emulgiert:
a) Vitamin K und
b) eine Mischung aus Vitamin D und Vitamin A,
wobei man das Emulgieren der Komponenten a) und b) jeweils in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe
vornimmt, anschließend die Emulsionen mischt, homogenisiert und durch Verdünnung mit Wasser die Viskosität der Emulsionen auf 50 bis 300 cP einstellt und die Mischung für die Herstellung eines stabilen Vitamin-Trockenpulvers, gegebenenfalls in Gegenwart eines Überzugsmaterials, trocknet.

6. Verfahren zur Herstellung einer stabilen Vitamin-Emulsion oder eines stabilen Vitamin-Trockenpulvers **dadurch gekennzeichnet, daß** man
a) eine Mischung aus Vitamin A, Vitamin K und Vitamin D in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe in Wasser bei 50°C bis 70°C emulgiert,
die Emulsion homogenisiert und durch Verdünnung mit Wasser die Viskosität der Emulsion auf 50 bis 300 cP einstellt und für die Herstellung eines stabilen Vitamin-Trockenpulvers, gegebenenfalls in Gegenwart eines Überzugsmaterials, trocknet.

7. Verfahren nach den Ansprüchen 5 und 6 **dadurch gekennzeichnet, daß** das Vitamingemisch 70 bis 98 Gew.% Vitamin A, 0.01 bis 1 Gew.% Vitamin D und 2 bis 30 Gew.% Vitamin K enthält, wobei sich die Gew.%-Angaben der Einzelkomponenten zu 100% addieren.

8. Verfahren zur Herstellung einer stabilen Vitamin-Emulsion oder eines stabilen Vitamin-Trockenpulvers **dadurch gekennzeichnet, daß** man bei 50°C bis 70°C jeweils separat in Wasser emulgiert:
a) Vitamin K und
b) eine Mischung aus Vitamin A und Vitamin E,
wobei man das Emulgieren der Komponenten a) und b) jeweils in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe,
vornimmt, anschließend die Emulsionen mischt, homogenisiert und durch Verdünnung mit Wasser die Viskosität der Emulsionen auf 50 bis 300 cP einstellt und die Mischung für die Herstellung eines stabilen Vitamin-Trockenpulvers, gegebenenfalls in Gegenwart eines Überzugsmaterials, trocknet.

9. Verfahren zur Herstellung einer stabilen Vitamin-Emulsion oder eines stabilen Vitamin-Trockenpulvers **dadurch gekennzeichnet, daß** man
a) eine Mischung aus Vitamin A, Vitamin E und Vitamin K in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe
in Wasser bei 50°C bis 70°C emulgiert, die Emulsion homogenisiert und durch Verdünnung mit Wasser die Viskosität der Emulsion auf 50 bis 300 cP einstellt und für die Herstellung eines stabilen Vitamin-Trockenpulvers, gegebenenfalls in Gegenwart eines Überzugsmaterials, trocknet.

10. Verfahren nach den Ansprüchen 8 und 9 **dadurch gekennzeichnet, daß** das Vitamingemisch 5 bis 90 Gew.% Vitamin A, 5 bis 90 Gew.% Vitamin E und 0.1 bis 10 Gew.% Vitamin K enthält, wobei sich die Gew.%-Angaben der Einzelkomponenten zu 100% addieren.

11. Verfahren zur Herstellung einer stabilen Vitamin-Emulsion oder eines stabilen Vitamin-Trockenpulvers **dadurch gekennzeichnet, daß** man eine Mischung aus
a) Vitamin D, Vitamin E sowie Vitamin A oder einem inerten Öl oder eine Mischung aus Vitamin A und einem inerten Öl, erhältlich, indem man
a₁) Vitamin D in einem inerten Öl und/oder Vitamin A bei einer Temperatur zwischen 40°C bis 80°C löst und mit Vitamin E mischt oder
a₂) eine Mischung aus Vitamin E und Vitamin A und/oder einem inerten Öl mit Vitamin D versetzt, in Gegenwart
c) eines oder mehrerer Schutzkolloide,
d) gegebenenfalls eines oder mehrerer Zucker und/oder Zuckeralkohole und
e) gegebenenfalls weiterer Zusatzstoffe
in Wasser bei 50°C bis 70°C emulgiert, die Emulsion homogenisiert und durch Verdünnung mit Wasser die Viskosität der Emulsionen auf 50 bis 300 cP einstellt und für die Herstellung eines stabilen Vitamin-Trockenpulvers, gegebenenfalls in Gegenwart eines Überzugsmaterials, trocknet.

12. Verfahren nach Anspruch 11 **dadurch gekennzeichnet, daß** das Vitamingemisch 5 bis 90 Gew.% Vitamin A und/oder ein inertes Öl, 5 bis 90 Gew.%·Vitamin E und 0.01 bis 1 Gew.% Vitamin D enthält, wobei sich die Gew.%-Angaben der Einzelkomponenten zu 100% addieren.

13. Verfahren nach den Ansprüchen 1 bis 12 **dadurch gekennzeichnet, daß** man als Schutzkolloid: Gelatine, Gummi Arabicum, Pflanzenproteine und/oder modifizierte Stärke, als Zucker: Saccharose, Laktose und/oder Maltodextrin und als Überzugsmaterial: Maisstärke und/oder Reisstärke verwendet.

14. Verfahren nach den Ansprüchen 1 bis 13 **dadurch gekennzeichnet, daß** die Bezeichnung "Vitamin A, D, E und/oder K" auch deren Derivate und/oder Mischungen derselben beinhaltet.

15. Stabile Vitamin-Emulsion oder stabiles Vitamin-Trockenpulver erhältlich nach Verfahren gemäß den Ansprüchen 1 oder 2.

16. Stabile Vitamin-Emulsion oder stabiles Vitamin-Trockenpulver erhältlich nach Verfahren gemäß den Ansprüchen 5 oder 6.

17. Stabile Vitamin-Emulsion oder stabiles Vitamin-Trockenpulver erhältlich nach Verfahren gemäß den Ansprüchen 8 oder 9.

18. Stabile Vitamin-Emulsion oder stabiles Vitamin-Trockenpulver erhältlich nach dem Verfahren gemäß dem Anspruch 11.

19. Verwendung von stabilen Vitamin-Emulsionen und/oder Vitamin-Trockenpulvern gemäß den Ansprüchen 15 bis 18 als·Zusatz zu Lebensmitteln, Pharmazeutika und/oder Tierfuttermitteln.

20. Lebensmittel, Pharmazeutika und/oder Tierfuttermittel, enthaltend stabile Vitamin-Emulsionen und/oder stabile Vitamin-Trockenpulver gemäß den Ansprüchen 15 bis 18.

## Claims

1. A process for preparing a stable vitamin emulsion or a stable vitamin dry powder which comprises emulsifying, at from 50°C to 70°C, in each case separately in water:
a) vitamin K and
b) a mixture of vitamin D, vitamin E and vitamin A or an inert oil or a mixture of vitamin A and an inert oil, obtainable by
b₁) dissolving vitamin D in an inert oil and/or vitamin A at a temperature between 40°C and 80°C, and mixing with vitamin E, or
b₂) adding vitamin D to a mixture of vitamin E and vitamin A and/or an inert oil,
carrying out the emulsification of components a) and b) in each case in the presence
c) of one or more protective colloids,
d) where appropriate of one or more sugars and/or sugar alcohols and
e) where appropriate other additives,
subsequently mixing the emulsions, homogenizing them and adjusting the viscosity of the emulsions to 50 to 300 cP by dilution with water, and drying the mixture to prepare a stable vitamin dry powder, where appropriate in the presence of a coating material.

2. A process for preparing a stable vitamin emulsion or a stable vitamin dry powder which comprises emulsifying a mixture of
a) vitamin D, vitamin E and vitamin K, and vitamin A or an inert oil or a mixture of vitamin A and an inert oil, obtainable by
a₁) dissolving vitamin D in an inert oil and/or vitamin A at a temperature between 40°C and 80°C, and mixing with vitamin E and vitamin K, or
a₂) adding vitamin D to a mixture of vitamin E and vitamin K, and vitamin A and/or an inert oil,
in the presence
c) of one or more protective colloids,
d) where appropriate of one or more sugars and/or sugar alcohols and
e) where appropriate of other additives,
in water at from 50°C to 70°C, homogenizing the emulsion and adjusting the viscosity of the emulsions to 50 to 300 cP by dilution with water, and drying to prepare a stable vitamin dry powder, where appropriate in the presence of a coating material.

3. A process as claimed in claims 1 and 2, wherein the vitamin mixture contains 5 to 90% by weight of vitamin A and/or an inert oil, 5 - 90 % by weight of vitamin E, 0.01 - 1% by weight of vitamin D and 0.1 - 10% by weight of vitamin K, where the % by weight data for the individual components add up to 100%.

4. A process as claimed in any of claims 1 to 3, wherein the vitamin mixture contains 25 - 70% by weight of vitamin A and/or an inert oil, 30 - 70% by weight of vitamin E, 0.05 - 0.4% by weight of vitamin D and 2 - 8% by weight of vitamin K, where the % by weight data for the individual components add up to 100%.

5. A process for preparing a stable vitamin emulsion or a stable vitamin dry powder which comprises emulsifying, at from 50°C to 70°C, in each case separately in water:
a) vitamin K and
b) a mixture of vitamin D and vitamin A, carrying out the emulsification of components a) and b) in each case in the presence
c) of one or more protective colloids,
d) where appropriate of one or more sugars and/or sugar alcohols and
e) where appropriate of other additives,
subsequently mixing the emulsions, homogenizing them and adjusting the viscosity of the emulsions to 50 to 300 cP by dilution with water, and drying the mixture to prepare a stable vitamin dry powder, where appropriate in the presence of a coating material.

6. A process for preparing a stable vitamin emulsion or a stable vitamin dry powder which comprises emulsifying
a) a mixture of vitamin A, vitamin K and vitamin D in the presence
c) of one or more protective colloids,
d) where appropriate of one or more sugars and/or sugar alcohols and
e) where appropriate of other additives in water at from 50°C to 70°C,
homogenizing the emulsion and adjusting the viscosity of the emulsion to 50 to 300 cP by dilution with water, and drying to prepare a stable vitamin dry powder, where appropriate in the presence of a coating material.

7. A process as claimed in claims 5 and 6 wherein the vitamin mixture contains 70 - 98% by weight of vitamin A, 0.01 -1% by weight of vitamin D and 2 - 30% by weight of vitamin K, where the % by weight data for the individual components add up to 100%.

8. A process for preparing a stable vitamin emulsion or a stable vitamin dry powder which comprises emulsifying, at from 50°C to 70°C, in each case separately in water:
a) vitamin K and
b) a mixture of vitamin A and vitamin E, carrying out the emulsification of components a) and b) in each case in the presence
c) of one or more protective colloids,
d) where appropriate of one or more sugars and/or sugar alcohols and
e) where appropriate of other additives, subsequently mixing the emulsions, homogenizing them and adjusting the viscosity of the emulsions to 50 to 300 cP by dilution with water, and drying the mixture to prepare a stable vitamin dry powder, where appropriate in the presence of a coating material.

9. A process for preparing a stable vitamin emulsion or a stable vitamin dry powder which comprises emulsifying
a) a mixture of vitamin A, vitamin E and vitamin K in the presence
c) of one or more protective colloids,
d) where appropriate of one or more sugars and/or sugar alcohols and
e) where appropriate of other additives, in water at from 50°C to 70°C, homogenizing the emulsion and adjusting the viscosiy of the emulsion to 50 to 300 cP by dilution with water, and drying to prepare a stable vitamin dry powder, where appropriate in the presence of a coating material.

10. A process as claimed in claims 8 and 9 wherein the vitamin mixture contains 5 - 90% by weight of vitamin A, 5 - 90% by weight of vitamin E and 0.1 - 10% by weight of vitamin K, where the % by weight data for the individual components add up to 100%.

11. A process for preparing a stable vitamin emulsion or a stable vitamin dry powder which comprises emulsifying a mixture of
a) vitamin D, vitamin E and vitamin A or an inert oil or a mixture of vitamin A and an inert oil, obtainable by
a₁) dissolving vitamin D in an inert oil and/or vitamin A at a temperature between 40°C and 80°C, and mixing with vitamin E, or
a₂) adding vitamin D to a mixture of vitamin E and vitamin A and/or an inert oil, in the presence
c) of one or more protective colloids,
d) where appropriate of one or more sugars and/or sugar alcohols and
e) where appropriate other additives, in water at from 50°C to 70°C, homogenizing the emulsion and adjusting the viscosity of the emulsions to 50 to 300 cP by dilution with water, and drying to prepare a stable vitamin dry powder, where appropriate in the presence of a coating material.

12. A process as claimed in claim 11, wherein the vitamin mixture contains 5 - 90% by weight of vitamin A and/or an inert oil, 5 - 90% by weight of vitamin E and 0.01 -1% by weight of vitamin D, where the % by weight data for the individual components add up to 100%.

13. A process as claimed in any of claims 1 to 12 wherein gelatin, gum arabic, vegetable proteins and/or modified starch is used as protective colloid, sucrose, lactose and/or maltodextrin is used as sugar, and corn starch and/or rice starch is used as coating material.

14. A process as claimed in any of claims 1 to 13, wherein the term "vitamin A, D, E and/or K" also comprises their derivatives and/or mixtures thereof.

15. A stable vitamin emulsion or stable vitamin dry powder obtainable by processes as claimed in claim 1 or 2.

16. A stable vitamin emulsion or stable vitamin dry powder obtainable by processes as claimed in claim 5 or 6.

17. A stable vitamin emulsion or stable vitamin dry powder obtainable by processes as claimed in claim 8 or 9.

18. A stable vitamin emulsion or stable vitamin dry powder obtainable by the process as claimed in claim 11.

19. The use of stable vitamin emulsions and/or vitamin dry powders as claimed in claims 15 to 18 as additives to human foods, drugs and/or animal feeds.

20. A human food, drug and/or animal feed comprising stable vitamin emulsions and/or stable vitamin dry powders as claimed in any of claims 15 to 18.

## Revendications

1. Procédé pour la préparation d'un émulsion stable de vitamines ou d'une poudre sèche de vitamines stable, **caractérisé par le fait qu'**on émulsifie entre 50°C et 70°C, à chaque fois séparément, dans l'eau:
a) de la vitamine K et
b) un mélange pouvant être obtenu à partir de vitamine D, de vitamine E et de vitamine A, ou d'une huile inerte, ou un mélange pouvant être obtenu à partir de vitamine A et d'une huile inerte, tandis que
b₁) on dissout la vitamine D dans une huile inerte et/ou la vitamine A à une température entre 40°C et 80°C et on mélange avec la vitamine E ou
b₂) on ajoute la vitamine D à un mélange de vitamine E et de vitamine A et/ou d'une huile inerte,
tandis qu'on conduit l'émulsification des composants a) et b) à chaque fois en présence de
c) un ou plusieurs colloïdes protecteurs,
d) éventuellement un ou plusieurs sucres et/ou alcools de sucre et
e) éventuellement d'autres additifs,
on mélange ensuite les émulsions, on homogénéise et on ajuste la viscosité des émulsions entre 50 et 300 cP par dilution avec de l'eau et, pour la préparation d'une poudre sèche de vitamines stable, on sèche le mélange, éventuellement en présence d'une matière d'enrobage.

2. Procédé pour la préparation d'une émulsion stable de vitamines ou d'une poudre sèche de vitamines stable, **caractérisé par le fait qu'**on émulsifie dans l'eau entre 50°C et 70°C un mélange
a) de vitamine D, de vitamine E et de vitamine K, ainsi que de vitamine A ou d'une huile inerte, ou un mélange pouvant être obtenu à partir de vitamine A et d'une huile inerte,
a₁) en dissolvant la vitamine D dans une huile inerte et/ou la vitamine A à une température entre 40°C et 80°C et en mélangeant avec la vitamine E et la vitamine K, ou
a₂) additionnant un mélange de vitamine E et de vitamine K, ainsi que de vitamine A et/ou d'une huile inerte avec la vitamine D,
en présence de
c) un ou plusieurs colloïdes protecteurs,
d) éventuellement un ou plusieurs sucres et/ou alcools de sucre et
e) éventuellement d'autres additifs,
on homogénéise l'émulsion et on ajuste la viscosité des émulsions entre 50 et 300 cP par dilution avec de l'eau et, pour la préparation d'une poudre sèche de vitamines stable, on sèche, éventuellement en présence d'une matière d'enrobage.

3. Procédé selon les revendications 1 et 2, **caractérisé par le fait que** le mélange de vitamines contient 5 à 90 % en poids de vitamine A et/ou d'une huile inerte, 5 à 90 % en poids de vitamine E, 0,01 à 1 % en poids de vitamine D et 0,1 à 10 % en poids de vitamine K, tandis que la somme des indications en % en poids des composants individuels est de 100 %.

4. Procédé selon les revendications 1 à 3, **caractérisé par le fait que** le mélange de vitamines contient 25 à 70 % en poids de vitamine A et/ou d'une huile inerte, 30 à 70 % en poids de vitamine E, 0,05 à 0,4 % en poids de vitamine D et 2 à 8 % en poids de vitamine K, tandis que la somme des indications en % en poids des composants individuels est de 100 %.

5. Procédé pour la préparation d'un émulsion stable de vitamines ou d'une poudre sèche de vitamines stable, **caractérisé par le fait qu'**on émulsifie entre 50°C et 70°C, à chaque fois séparément, dans l'eau:
a) de la vitamine K et
b) un mélange de vitamine D et de vitamine A, tandis qu'on conduit l'émulsification des composants a) et b) à chaque fois en présence de
c) un ou plusieurs colloïdes protecteurs,
d) éventuellement un ou plusieurs sucres et/ou alcools de sucre et
e) éventuellement d'autres additifs,
on mélange ensuite les émulsions, on homogénéise et on ajuste la viscosité des émulsions entre 50 et 300 cP par dilution avec de l'eau et, pour la préparation d'une poudre sèche de vitamines stable, on sèche le mélange, éventuellement en présence d'une matière d'enrobage.

6. Procédé pour la préparation d'une émulsion stable de vitamines ou d'une poudre sèche de vitamines stable, **caractérisé par le fait qu'**on émulsifie dans l'eau entre 50°C et 70°C
a) un mélange de vitamine A, de vitamine K et de vitamine D, en présence de
c) un ou plusieurs colloïdes protecteurs,
d) éventuellement un ou plusieurs sucres et/ou alcools de sucre et
e) éventuellement d'autres additifs,
on homogénéise l'émulsion et on ajuste la viscosité de l'émulsion entre 50 et 300 cP par dilution avec de l'eau et, pour la préparation d'une poudre sèche de vitamines stable, on sèche, éventuellement en présence d'une matière d'enrobage.

7. Procédé selon les revendications 5 et 6, **caractérisé par le fait que** le mélange de vitamines contient 70 à 98 % en poids de vitamine A, 0,01 à 1 % en poids de vitamine D et 2 à 30 % en poids de vitamine K, tandis que la somme des indications en % en poids des composants individuels est de 100 %.

8. Procédé pour la préparation d'un émulsion stable de vitamines ou d'une poudre sèche de vitamines stable, **caractérisé par le fait qu'**on émulsifie entre 50°C et 70°C, à chaque fois séparément, dans l'eau:
a) de la vitamine K et
b) un mélange de vitamine A et de vitamine E, tandis qu'on conduit l'émulsification des composants a) et b) à chaque fois en présence de
c) un ou plusieurs colloïdes protecteurs,
d) éventuellement un ou plusieurs sucres et/ou alcools de sucre et
e) éventuellement d'autres additifs, on mélange ensuite les émulsions, on homogénéise et on ajuste la viscosité des émulsions entre 50 et 300 cP par dilution avec de l'eau et, pour la préparation d'une poudre sèche de vitamines stable, on sèche le mélange, éventuellement en présence d'une matière d'enrobage.

9. Procédé pour la préparation d'une émulsion stable de vitamines ou d'une poudre sèche de vitamines stable, **caractérisé par le fait qu'**on émulsifie dans l'eau entre 50°C et 70°C
a) un mélange de vitamine A, de vitamine E et de vitamine K, en présence de
c) un ou plusieurs colloïdes protecteurs,
d) éventuellement un ou plusieurs sucres et/ou alcools de sucre et
e) éventuellement d'autres additifs,
on homogénéise l'émulsion et on ajuste la viscosité de l'émulsion entre 50 et 300 cP par dilution avec de l'eau et, pour la préparation d'une poudre sèche de vitamines stable, on sèche, éventuellement en présence d'une matière d'enrobage.

10. Procédé selon les revendications 8 et 9, **caractérisé par le fait que** le mélange de vitamines contient 5 à 90 % en poids de vitamine A, 5 à 90 % en poids de vitamine E et 0,1 à 10 % en poids de vitamine K, tandis que la somme des indications en % en poids des composants individuels est de 100 %.

11. Procédé pour la préparation d'une émulsion stable de vitamines ou d'une poudre sèche de vitamines stable, **caractérisé par le fait qu'**on émulsifie dans l'eau entre 50°C et 70°C un mélange.
a) de vitamine D, de vitamine E, ainsi que de vitamine A ou d'une huile inerte, ou un mélange pouvant être obtenu à partir de vitamine A et d'une huile inerte,
a₁) en dissolvant la vitamine D dans une huile inerte et/ou la vitamine A à une température entre 40°C et 80°C et en mélangeant avec la vitamine E, ou
a₂) additionnant un mélange de vitamine E et de vitamine A et/ou d'une huile inerte avec la vitamine D,
en présence de
c) un ou plusieurs colloïdes protecteurs,
d) éventuellement un ou plusieurs sucres et/ou alcools de sucre et
e) éventuellement d'autres additifs, on homogénéise l'émulsion et on ajuste la viscosité des émulsions entre 50 et 300 cP par dilution avec de l'eau et, pour la préparation d'une poudre sèche de vitamines stable, on sèche, éventuellement en présence d'une matière d'enrobage.

12. Procédé selon la revendication 11, **caractérisé par le fait que** le mélange de vitamines contient 5 à 90 % en poids de vitamine A et/ou d'une huile inerte, 5 à 90 % en poids de vitamine E et 0,01 à 1 % en poids de vitamine D, tandis que la somme des indications en % en poids des composants individuels est de 100 %.

13. Procédé selon les revendications 1 à 12, **caractérisé par le fait qu'**on utilise comme colloïde protecteur: des gélatines, de la gomme arabique, des protéines végétales et/ou des amidons modifiés, comme sucre: du saccharose, du lactose et/ou de la maltodextrine, et comme matière d'enrobage: de l'amidon de maïs et/ou de l'amidon de riz;

14. Procédé selon les revendications 1 à 13, **caractérisé par le fait que** la désignation "vitamine A, D, E et/ou K" englobe également leurs dérivés et/ou des mélanges de ceux-ci.

15. Emulsion stable de vitamines ou poudre sèche de vitamines stable pouvant être obtenue conformément au procédé selon les revendications 1 ou 2.

16. Emulsion stable de vitamines ou poudre sèche de vitamines stable pouvant être obtenue conformément au procédé selon les revendications 5 ou 6.

17. Emulsion stable de vitamines ou poudre sèche de vitamines stable pouvant être obtenue conformément au procédé selon les revendications 8 ou 9.

18. Emulsion stable de vitamines ou poudre sèche de vitamines stable pouvant être obtenue conformément au procédé selon la revendication 11.

19. Utilisation d'émulsions stables de vitamines et/ou de poudres sèches de vitamines stables selon les revendications 15 à 18 comme additif pour produits alimentaires, produits pharmaceutiques et/ou aliments pour animaux.

20. Produits alimentaires, produits pharmaceutiques et/ou aliments pour animaux, contenant de émulsions stables de vitamines et/ou des poudres sèches de vitamines stables selon les revendications 15 à 18.
